# EUROPEAN PATENT APPLICATION

(11) **EP 2 320 293 A1**
(43) Date of publication of application: **11.05.2011**
(21) Application number: 09175109.9
(22) Date of filing: 05.11.2009
(51) Int. Cl.: G05D 23/19

(54) **Sleep element for improving the sleep of a person**

(71) Applicant: Koninklijke Philips Electronics N.V., 5621 BA Eindhoven (NL)
(72) Inventor: The designation of the inventor has not yet been filed
(74) Representative: Golla-Franz, Anke Lucia

(57) **Abstract**

The invention relates to a sleep element (1) like a heating blanket for improving the sleep of a person. The sleep element comprises a property determination unit (2, 5) for determining a property of a person, a thermal energy unit (3) for transferring thermal energy to or away from the person, and a thermal energy control unit (4) for controlling the thermal energy unit depending on the determined property of the person. Since the sleep element comprises the property determination unit, the thermal energy unit, and the thermal energy control unit, the temperature of the person can be controlled depending on a determined property of the person, while the person is sleeping, such that the sleep of the person is improved. Moreover, the temperature control can simply be used within a bed by placing the sleep element in the bed. The handling of the temperature control is therefore very simple.

## Description

### FIELD OF THE INVENTION

The invention relates to a sleep element, a sleep method and a sleep computer program for improving the sleep of a person. The invention relates further to a bed comprising the sleep element.

### BACKGROUND OF THE INVENTION

JP 04-295533 discloses a temperature controller for controlling the temperature within a bed. The temperature controller comprises a pyroelectric type infrared sensor being installed at a frame of the bed above a site at which the head of a person is to be located. The infrared sensor is adapted to detect the movement of the person in the bed. The temperature controller further comprises a control circuit that is also installed at the frame of the bed above the site at which the head of the person is to be installed. The control circuit is connected to the infrared sensor and counts the number of signals generated by the infrared sensor per specified time period. A temperature regulator located below the mattress of the bed is controlled according to the number of signals counted by the control circuit.

The handling of the temperature controller is not simple, because an infrared sensor and a controller have to be installed at the bed frame and a temperature regulator has to be located below the mattress. If the temperature controller has to be used with another bed, the infrared sensor, the control circuit, and the temperature regulator have to be detached from the bed and again installed at the other bed.

### SUMMARY OF THE INVENTION

It is an object of the present invention to provide an apparatus, which allows controlling the temperature of a person lying in a bed, wherein the handling of the apparatus is simplified.

In a first aspect of the present invention a sleep element for improving the sleep of a person is presented, wherein the sleep element being adapted to be used within a bed and comprises:
- a property determination unit for determining a property of a person,
- a thermal energy unit for transferring thermal energy to or away from the person,
- a thermal energy control unit for controlling the thermal energy unit depending on the determined property of the person.

The sleep element is preferentially a blanket, a pillow, or a mattress. Since the sleep element comprises a property determination unit for determining a property of a person, a thermal energy unit for transferring thermal energy to or away from the person, and a thermal energy control unit for controlling the thermal energy unit depending on the determined property of the person, the temperature of the person can be controlled depending on a determined property of the person, while the person is sleeping. This control of the temperature can be used for improving the sleep of the person. Moreover, since the sleep element comprises the different units used for controlling the temperature of the person, the temperature control can simply be used within a bed by placing the sleep element like a blanket in the bed. If the temperature control used with a certain bed should be used with another bed, the sleep element has just to be moved from the certain bed to the other bed. For example, a blanket being the sleep element has just to be moved from the certain bed to the other bed. The handling of the temperature control is therefore very simple.

It is preferred that the property determination unit and the thermal energy unit are integrated within the sleep element.

The property determination unit and the thermal energy unit can be integrated completely within the sleep element or only parts of these units can be integrated within the sleep element. In particular, the sleep element is preferentially a blanket, wherein the property determination unit and the thermal energy unit are integrated within the blanket.

It is further preferred that the property determination unit comprises a moving sensor for generating a movement signal being indicative of a movement of the person and a comfort level determining unit for determining a comfort level of the person as the property of the person depending on the generated movement signal. This allows controlling the temperature of the person depending on the comfort level of the person.

The moving sensor comprises preferentially an accelerometer for generating the movement signal. The comfort level determining unit can be adapted to determine a comfort level of the person depending on the amplitude and/or frequency of the movement signal.

It is further preferred that the comfort level determining unit is adapted to provide movement - comfort level assignments between movement signals and comfort levels and to determine a comfort level depending on the generated movement signal based on the provided movement - comfort level assignments. This allows determining the comfort level in a simple way by simply using the movement-comfort level assignments and the generated movement signal. The assignments can be stored as thresholds defining regions in a movement signal space, wherein, if a generated movement signal is within a certain region of the movement signal space, a comfort level is determined, which has been assigned to this certain region. The thresholds can be stored in the comfort level determining unit. The thresholds are, for example, thresholds for the amplitude and/or the frequency of the generated movement signal. The movement-comfort level assignments can also be provided as one or several functions having as an input the generated movement signal and as an output a comfort level.

It is further preferred that the comfort level determining unit is adapted to provide a movement - comfort level assignment of a movement signal to a comfort level indicating an uncomfortable condition by providing an uncomfortable threshold, wherein the comfort level determining unit is adapted to determine the comfort level indicating an uncomfortable condition, if the generated movement signal exceeds the uncomfortable threshold. This allows controlling the temperature of the person such that the person feels comfortable by using just one threshold.

It is further preferred that the thermal control unit is adapted to determine a thermal energy pattern defining the transferring of thermal energy to or away from the person depending on the determined comfort level and to control the thermal energy unit such that the transferring of thermal energy to or away from the person is performed depending on the determined thermal energy pattern.

The thermal energy pattern preferentially defines the amount of thermal energy which has to be transferred to or away from the person. The thermal energy pattern can further define the positions at which the thermal energy should be transferred to or away from the person. The thermal energy pattern can also define the times at which the thermal energy should be transferred to or away from the person.

It is further preferred that the thermal energy control unit is adapted to provide comfort level - thermal energy assignments between comfort levels and thermal energy patterns and to determine a thermal energy pattern depending on the determined comfort level based on the comfort level - thermal energy assignments.

It is further preferred that the thermal energy control unit is adapted to determine a thermal energy pattern defining an additional transfer of thermal energy to the person, if the comfort level determining unit determines a comfort level indicating an uncomfortable condition.

It is further preferred that the thermal energy unit comprises a thermo-electric element for electrically transferring thermal energy to or away from the person by heating or cooling the person. This allows heating or cooling the person for controlling the temperature of the person in a simple way. In an embodiment, the same thermo-electric element can be used for heating or cooling the person, wherein for switching from heating to cooling or vice versa the direction of the current flowing through the thermo-electric element is modified. Preferentially, the thermal energy unit comprises several thermo-electric elements arranged at different locations for allowing the person to be heated or cooled at different locations differently. For example, the thermo-electric elements can be distributed homogenously over the sleep element like a blanket, wherein, for example, it is possible to transfer more heat to the feet of the person than to other body parts of the person. Thus, preferentially single thermo-electric elements and/or groups of thermo-electric elements are separately addressable.

It is further preferred that the thermal energy unit comprises an electrically conducting element, wherein the property determining unit is adapted to measure an electrical property of the electrically conducting element and to determine a property of the person depending on the measured electrical property. The thermal energy unit can comprise one or several electrically conducting elements.

The thermal energy unit and the property determining unit can therefore highly be integrated. Moreover, for example, sleep elements with a standard thermal energy unit like blankets with a standard thermal energy unit can be used, without modifying the elements within the sleep element. It is just required to connect a measurement unit for measuring an electrical property of the electrically conducting elements of the thermal energy unit to these electrically conducting elements for determining a property of the person. The electrically conducting elements are preferentially electrical wires within the sleep element used themselves for heating or for transferring electrical energy to another thermo-electric element. The electrical property being measured by the property determining unit is, for example, the resistance, capacitance and/or the inductance of the electrically conducting elements. If the person moves and if the person is in contact with the sleep element, for example, if the sleep element is a blanket and the person lies on the blanket, the geometry of the different elements of the sleep element is changed. This geometry change generally leads to a change of, for example, the capacitance and/or the inductance of the electrical conducting elements. Thus, by measuring an electrical property of the electrically conducting elements a signal can be generated being indicative of a movement of the person. The measured electrical property of the electrically conducting elements can therefore be regarded as a movement signal.

It is further preferred that the sleep element comprises a temperature sensor for measuring the temperature of the sleep element, wherein the thermal energy control unit is adapted to control the thermal energy unit such that the thermal energy transferred to the person is lowered, if the temperature sensor measures a temperature exceeding a temperature threshold. The temperature threshold is preferentially chosen such that an overheating of the sleep element is prevented. Preferentially, the temperature threshold is 40 degree Celsius or smaller.

It is further preferred that the sleep element comprises:
- a property recording unit for recording properties of the person, which have been determined during sleeping, the recorded properties of the person forming a recorded property pattern,
- a user interface for allowing the person to select between at least good sleep quality and bad sleep quality,
- an assignment generation unit for generating assignments between the recorded property pattern and the selected sleep quality and for determining a reference property pattern depending on the generated assignments,
wherein the thermal control unit is adapted to control the thermal energy unit such that a deviation of actually determined properties of the person forming an actual property pattern and the reference property pattern is reduced.

This allows a user to adapt the temperature control to his personal preferences. The temperature control can therefore be adapted to the respective user. The property of the person recorded by the property recording unit is, for example, the skin temperature of the person. In this case, the property determination unit comprises therefore a temperature sensor for measuring the skin temperature of the person. However, the property recording unit can also be adapted to record another property of the person like a movement of the person by recording the movement signal.

The user interface has, for example, two buttons, a red one and a green one. If the sleep was good, the person can press the green button, and if the sleep was bad, the user can press the red button. The user interface can also be adapted to select between more than two quality levels. For example, the user interface can be adapted to select between good sleep quality, mid-level sleep quality, and bad sleep quality. The user interface can comprise a yellow button for allowing a user to select a mid-level sleep quality.

A reference property pattern can define the maximal and/or minimal determined property value, in particular, the maximal and/or minimal skin temperature or movement signal. The thermal control unit can be adapted such that the actual skin temperature or movement signal determined while the person is sleeping is above the minimal skin temperature or movement signal and below the maximal skin temperature or movement signal, wherein these minimal and maximal values have been rated by the user as providing a good sleep quality.

In a further aspect of the present invention a bed is presented, wherein the bed comprises the sleep element as defined in claim 1.

In a further aspect of the present invention a sleep method for improving the sleep of a person is presented, wherein a sleep element for being used within a bed as defined in claim 1 is provided and wherein the sleep method comprises following steps:
- determining a property of a person by the property determination unit,
- transferring thermal energy to or away from the person by the thermal energy unit, wherein the transferring of the thermal energy to or away from the person is controlled depending on the determined property of the person by the thermal energy control unit.

In a further aspect of the presesent invention a sleep computer program for improving the sleep of a person is presented, wherein the sleep computer program comprises program code means for causing a sleep element as defined in claim 1 to carry out the steps of the sleep method as defined in claim 14, when the sleep computer program is run on a computer controlling the sleep element.

It shall be understood that the sleep element of claim 1, the bed of claim 13, the sleep method of claim 14 and the computer program of claim 15 have similar and/or identical preferred embodiments as defined in the dependent claims.

It shall be understood that a preferred embodiment of the invention can also be any combination of the dependent claims with the respective independent claim.

These and other aspects of the invention will be apparent from and elucidated with reference to the embodiments described hereinafter.

### BRIEF DESCRIPTION OF THE DRAWINGS

In the following drawings:
- Fig. 1: shows schematically and exemplarily an embodiment of a sleep element for improving the sleep of a person,
- Fig. 2: shows schematically and exemplarily a further embodiment of a sleep element for improving the sleep of a person,
- Fig. 3: shows schematically and exemplarily a further embodiment of a sleep element for improving the sleep of a person,
- Fig. 4: shows schematically and exemplarily a further embodiment of a sleep element for improving the sleep of a person,
- Fig. 5: shows a flowchart exemplarily illustrating an embodiment of a sleep method for improving the sleep of a person, and
- Fig. 6: shows a flowchart exemplarily illustrating a further embodiment of a sleep method for improving the sleep of a person.

### DETAILED DESCRIPTION OF EMBODIMENTS

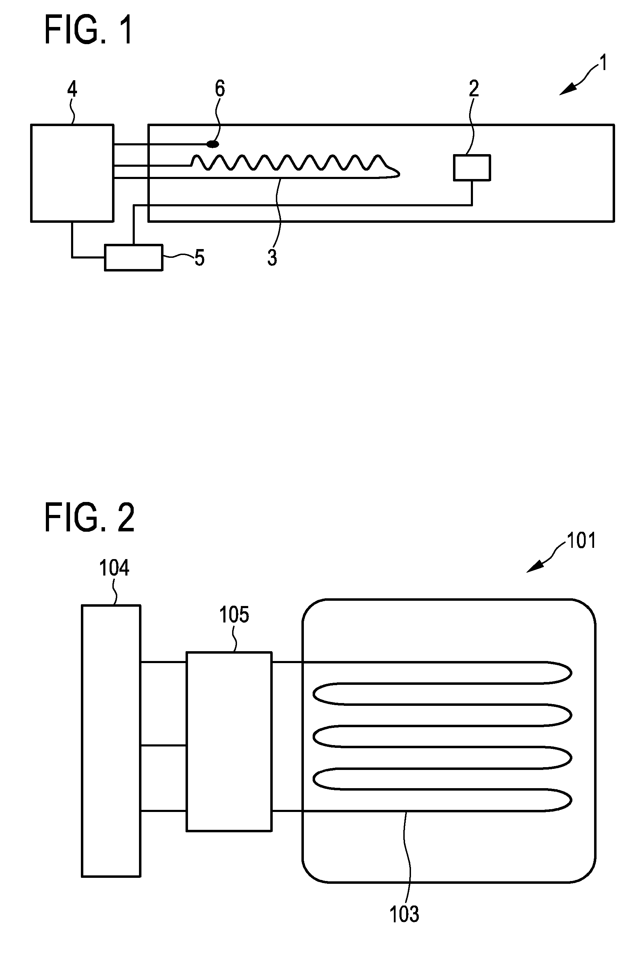

Fig. 1 shows schematically and exemplarily a sleep element 1 for improving the sleep of a person. In this embodiment, the sleep element 1 is a blanket for being used within a bed. The sleep element 1 comprises an accelerometer 2 for detecting movements of a person, if the person lies on the sleep element 1 or is covered by the sleep element 1. The accelerometer 2 is therefore a moving sensor for generating a movement signal being indicative of a movement of a person.

The sleep element 1 further comprises a comfort level determining unit 5 for determining a comfort level of the person depending on the generated movement signal. The comfort level can be regarded as a property of the person and the combination of the moving sensor 2 and the comfort level determining unit 5 can be regarded as a property determination unit for determining a property of a person.

The sleep element 1 further comprises a heating element 3 being a thermal energy unit for transferring thermal energy to the person. The thermal energy unit 3 is a thermo-electric element for electrically transferring thermal energy to the person by heating. In particular, the thermo-electric element is a heating wire which heats the sleep element 1 and thus a person being in contact with the sleep element 1 via resistive heating. In another embodiment, the thermo-electric element can be adapted for electrically transferring thermal energy to and away from the person by heating and cooling the person, wherein for switching from heating to cooling or vice versa the direction of the current flowing through the thermo-electric element is modified. For example, the thermo-electric element can be a Peltier element which is based on the Peltier effect. The thermo-electric element can also be based on another effect like the Seeback effect or the Thomson effect. In particular, the thermo-electric element can also be a micro refrigerating element.

The thermal energy unit can comprise several thermo-electric elements arranged at different locations for allowing the person to be heated or cooled at different locations differently. For example, the thermo-electric elements can be distributed homogenously over the sleep element, in particular, over the blanket, wherein, for example, it is possible to transfer more heat to the feet of the person than to other body parts of the person. Single thermo-electric elements and/or groups of thermo-electric elements can be addressable separately.

The sleep element 1 further comprises a thermal energy control unit 4 for controlling the thermal energy unit 3 depending on the determined property of the person. The thermal energy control unit 4 comprises preferentially a power supply for modifying the voltage applied to the thermal energy unit 3 depending on the determined property of the person. The moving sensor 2 and the thermal energy unit 3 are integrated within the sleep element 1.

The comfort level determining unit 5 is adapted to determine a comfort level of the person depending on the amplitude and/or frequency of the movement signal, i.e. in this embodiment of the signal generated by the accelerometer 2.

The accelerometer 2 is preferentially an accelerometer of a piezoelectrical type. However, in another embodiment an accelerometer of another type can be used. The accelerometer can be adapted to measure the acceleration in one spatial axis, in two spatial axes or in three spatial axes. If the acceleration is measured in more than one spatial axis, the movement signal is preferentially a combination of the accelerometer signals measured for the different spatial axes. This combination is preferentially a linear combination, in particular, an average, of the accelerometer signals measured for different spatial axes.

In the comfort level determining unit 5 movement-comfort level assignments between amplitudes and/or frequencies of movement signals and comfort levels are stored, wherein the comfort level determining unit 5 is adapted to determine a comfort level depending on the generated movement signal, in particular, depending on the amplitude and/or frequency of the generated movement signal, based on the provided movement-comfort level assignments. The movement-comfort level assignments are defined by thresholds. The thresholds define regions in a movement signal space, wherein, if a generated movement signal is within a certain region of the movement signal space, a comfort level is determined which has been assigned to this certain region. The thresholds are, in this embodiment, thresholds for the amplitude and/or the frequency of the generated movement signal. Thus, a generated movement signal is classified in accordance with its amplitude and/or frequency and a comfort level is determined, which is assigned to the class in which the movement signal has been classified. In other embodiments, the movement-comfort level assignments can also be provided as one or several functions having as an input the generated movement signal and as an output a comfort level.

Preferentially, an uncomfortable threshold is stored in the comfort level determining unit 5. The uncomfortable threshold defines, for example, a certain amplitude and/or frequency of the movement signal, wherein, if the actually measured movement signal has an amplitude and/or frequency above the uncomfortable threshold, a comfort level is determined indicating an uncomfortable condition of the person, and, if the amplitude and/or frequency of the actually measured movement signal is below the uncomfortable threshold, a comfort level is determined indicating a comfortable condition. Thus, movement-comfort level assignments are preferentially stored in the comfort level determining unit 5 by storing the uncomfortable threshold. This allows controlling the temperature of a person such that the person feels comfortable by using just one threshold.

The thermal control unit 4 is adapted to determine a thermal energy pattern defining the transferring of thermal energy 2 to the person depending on the determined comfort level and to control the thermal energy unit 3 such that the transferring of thermal energy to the person is performed depending on the determined thermal energy pattern. The thermal energy pattern defines the amount of thermal energy which has to be transferred to the person. If the thermal energy unit comprises thermo-electric elements which also allow to cool the person, i.e. which allow to transfer thermal energy away from the person, the thermal energy pattern can define the amount of thermal energy which has to be transferred away from the person. The thermal energy pattern can further define the positions at which the thermal energy should be transferred to or away from the person. The thermal energy pattern can also define the times at which the thermal energy should be transferred to or away from the person.

The thermal energy control unit 4 is adapted to provide comfort level-thermal energy assignments between comfort levels and thermal energy patterns and to determine a thermal energy pattern depending on the determined comfort level based on the comfort level-thermal energy assignments. For example, at least two comfort levels can be defined, a first comfort level indicating that the person feels comfortable and a second comfort level indicating that the person feels uncomfortable. In an embodiment, to the first comfort level a first thermal energy pattern is assigned and to the second comfort level a second thermal energy pattern is assigned.

The thermal energy control unit 4 is preferentially adapted such that a thermal energy pattern defining an additional transfer of thermal energy to the person is determined, if the comfort level determining unit 5 determines the second comfort level, i.e. a comfort level indicating an uncomfortable condition of the person. Thus, in an embodiment, if the movement signal exceeds the uncomfortable threshold, which is preferentially preset, i.e. if the detected activity of the person exceeds the uncomfortable threshold, it is interpreted as an uncomfortable temperature setting and the thermal energy control unit 4 triggers the heating of the blanket 1 or adds some more power to the giving heating, if the blanket is already heating the person.

The sleep element 1 further comprises a temperature sensor 6 for measuring the temperature of the sleep element 1. The thermal energy control unit 4 is adapted to lower the thermal energy transferred to the person, if the temperature sensor 6 measures a temperature exceeding a temperature threshold. The temperature threshold is chosen such that an overheating of the sleep element is prevented. In particular, the thermal energy control unit is adapted to switch off the heating, if the temperature sensor 6 measures a temperature exceeding the temperature threshold. This protects a user from overheating. The temperature sensor 6 can be separated from the thermal energy unit 3, in particular, from the thermo-electric element, or the temperature sensor 6 can be attached to the thermal energy unit.

The sleep element 1 provides therefore a sensing mechanism to adjust to the actual user condition. In particular, the sleep element allows sensing a user condition for better assessment of human thermoregulation during sleep. Preferentially, it allows detecting user micro-movements as an indicator of uncomfortable cold or warm temperatures in bed. This indicator is preferentially used to control these temperatures. In an embodiment, in addition this indicator can be used to control air conditioning or other components of an indoor environment. The sleep element provides an unobtrusive way of sensing the user condition relevant to sleep thermoregulation. It does preferentially not require any skin, rectal or armpit temperature sensor. In an embodiment, the temperature sensor 6 can be omitted. In this case, the sleep element does not need any temperature sensor at all, because the actual condition of the user is determined by using the movement signal generated by the movement sensor which is, in the above described embodiment, an accelerometer. A direct contact with the skin or any other body part is then not needed.

Fig. 2 shows schematically and exemplarily a further embodiment of a sleep element. The sleep element 101 shown in Fig. 2 is also a blanket for being used within a bed. The sleep element 101 comprises a thermal energy unit 103 for transferring thermal energy to a person. In this embodiment, the thermal energy unit 103 is a thermo-electric element being an electrical wire for heating the blanket 101 by resistive heating. In other embodiments, the thermo-electric element can be adapted for heating and cooling purposes, wherein for switching between heating and cooling the direction of the current applied to the thermo-electric element is modified. The thermal energy unit 103 is controlled by a thermal energy control unit 104 depending on a determined property of the person. The thermal energy control unit 104 preferentially comprises a power supply for applying a voltage to the thermal energy unit 103.

The property determining unit 105 is adapted to measure an electrical property of the electrical wire of the thermal energy unit 103 being an electrically conducting element and to determine a property of the person depending on the measured electrical property. The conducting wire is distributed in a layer within the blanket 101, wherein the property determining unit 105 is preferentially adapted to measure the resistance, the capacitance or the inductance as the electrical property of the conducting wire. When a person moves in close proximity of the blanket 101, it changes the system geometry, i.e. it changes the geometry of the conducting wire and the environment. This change leads to a change of at least the capacitance and inductance, which can be measured by the property determining unit 105. Also the blanket geometry can be deformed from its original shape, resulting in an additional change in the electrical properties of the conducting wire. The property determining unit 105 is preferentially adapted to detect all these changes that are reflecting the movements of the person. The measured electrical property of the electrically conducting wire is therefore a movement signal. This movement signal can be used by the thermal energy control unit 104 for controlling the thermal energy unit 103 depending on the movements of the person. In particular, the property determination unit 105 can be adapted to determine a comfort level and the thermal energy control unit 104 can be adapted to control the thermal energy unit 103 depending on the determined comfort level as described above with reference to Fig. 1.

The sleep element 101 allows unobtrusively sensing a person in a bed in a simple way. The sleep element 101 can be realised with minor or no changes within the existing design of electrical blankets, i.e. no special sensors embedded into the blanket are required.

The conducting elements within the sleep element 101 can be coupled to each other directly for forming one heating wire, or they can be coupled by capacitive or inductive means. In the latter case the conducting elements are preferentially provided by wire segments. Thus, the thermal energy unit can comprise a single heating wire, or the thermal heating unit can comprise heating wire segments, which are preferentially capacitively and/or inductively coupled. The property determining unit can be adapted to measure the resistance, conductance, capacity and/or inductance of the conducting elements in a quantitative or comparative way.

The property determining unit can be adapted to determine a combined electrical property from the electrical properties measured for the different conducting elements. The combined electrical property is an electrical property which depends on the electrical properties of the different conducting elements. For example, the combined electrical property is a linear combination like an average or a maximum or minimum electrical property of the electrical properties measured for the different conducting elements. The combined electrical property can also be the result of a comparison of the electrical properties measured for the different conducting elements. For example, the combined electrical property can be the variance of the electrical properties measured for the different conducting elements. The combined electrical property can be compared with a reference value, wherein the reference value is preferentially a combined electrical property determined for a certain situation, for example, determined if the sleep element is used by a user and the user feels comfortable. The energy control unit 104 can be adapted to control the thermal energy unit 103 such that the combined electrical property is preferentially similar to the reference value. The sleep element 101 is preferentially adapted to allow a user to set the reference value.

Although in the above described embodiment the sleep element 101 determines a movement of the person depending on the measured electrical property, in other embodiments the measured electrical property can be used to measure another or a further property of the person. For example, the measured electrical property can be used for determining the skin temperature. Also the skin temperature modifies the electrical properties of the electrically conducting elements within the sleep element. This modification of the electrical properties can therefore also be indicative of the skin temperature of the person and can therefore be used to determine the skin temperature.

The electrical signal measured by the property determining unit 105 can also be regarded as a signal reflecting different properties of the person, for example, reflecting a movement of the person and a skin temperature of the person. In this case, the thermal energy control unit 105 controls the thermal energy unit 103 depending on movements of the person and the skin temperature of the person.

Although in the above described embodiment the electrically conductive elements are electrical wires, in other embodiments other flexible conductive elements can be used.

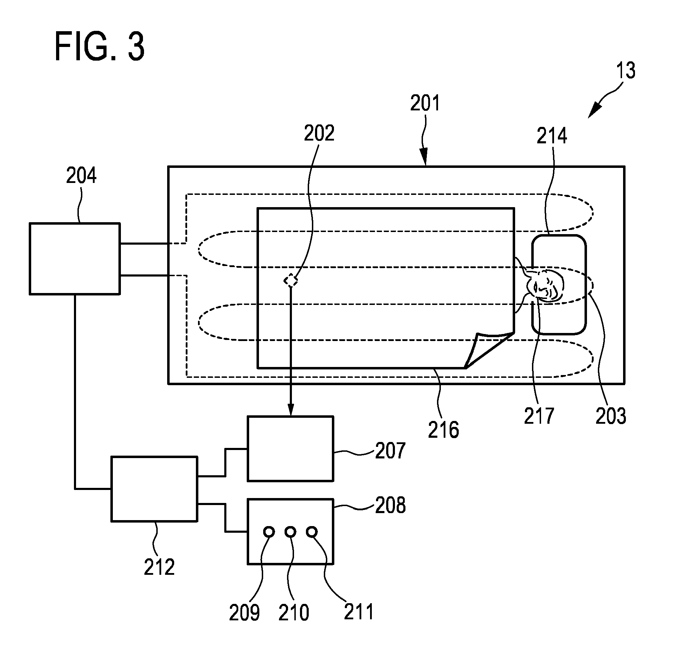

Fig. 3 shows schematically and exemplarily a further embodiment of a sleep element. The sleep element 201 is an electrical blanket, in particular, an electrical underblanket. The sleep element 201 is located on a mattress of a bed 13. A person 217 is lying on the electrical blanket 201. The head of the person 217 is located on a pillow 214 and the person 217 is covered by a covering blanket 216. The sleep element 201, i.e. in this embodiment the underblanket 201, comprises a property determination unit 202 being a skin temperature sensor. The skin temperature sensor 202 is embedded in the underblanket 201. The underblanket 201 comprises a thermal energy unit 203 being a thermo-electrical element. The thermo-electrical element is, in this embodiment, a heating wire for heating the underblanket 201 by resistive heating. The sleep element 201 further comprises a thermal energy control unit 204 for controlling the thermal energy unit 203 depending on the determined property of the person, i.e. in this embodiment depending on the measured skin temperature.

The sleep element 201 further comprises a property recording unit 207 for recording properties of the person 217, which have been determined during sleeping. In this embodiment, the property recording unit 217 is adapted to store the measured temperatures in a memory, while the person is sleeping. The recorded properties of the person 217 form a recorded property pattern. In this embodiment, the skin temperature of the person 217 is measured by the skin temperature sensor 202 and the skin temperature measured over time is stored in the property recording unit 207 as a recorded property pattern.

The sleep element 201 further comprises a user interface 208 for allowing a person to start the recording of the properties and to stop the recording of the properties. Preferentially, when a person wants to sleep, the person starts the recording of the properties, and if the person has waken up after having slept, the person 217 can stop the recording of the properties by using the interface 208.

The user interface 208 is further adapted for allowing the person 217 to select between good sleep quality, mid-level sleep quality and bad sleep quality. The user interface 208 comprises three buttons, a red one, a yellow one and a green one. If the sleep was good, the person can press the green button, if it was a mid-level sleep quality, the person can press the yellow button, and if the sleep was bad, the person can press the red button. These buttons are indicated in Fig. 3 by reference signs 209, 210, 211.

The sleep element 201 further comprises an assignment generation unit 212 for generating assignments between the recorded property pattern and the selected sleep quality and for determining a reference property pattern depending on the generated assignments. The thermal control unit 204 is adapted to control the thermal energy unit 203 such that a deviation of actually determined properties of the person 217 forming an actual property pattern and the reference property pattern is reduced. Thus, in this embodiment, the skin temperature is recorded while the person is sleeping. The skin temperature measured over time forms a recorded property pattern. After the person has rated the sleep via the user interface 208, the selected sleep quality is assigned to the recorded property pattern. The assignment generation unit 212 is preferentially adapted to combine several recorded property patterns which have been assigned to a sleep quality. This combining of several recorded property patterns is, for example, a weighted averaging, wherein a recorded property pattern assigned to good sleep quality receives a larger weight than a recorded property pattern assigned to mid-level sleep quality and wherein a recorded property pattern assigned to mid-level sleep quality receives a larger weight than a recorded property pattern assigned to bad sleep quality. The resulting combined recorded property pattern is the reference property pattern. The thermal control unit 204 controls the thermal energy unit 203 preferentially such that the reference property pattern, which is, in this embodiment, a measured skin temperature over time, is met by an actually measured property pattern, i.e. an actually measured skin temperature over time, as good as possible.

Thus, in this embodiment the weighting of the sleep quality is used to weight the stored skin temperature data, wherein, after the learning period is finished, the most optimal trend is selected to ensure the settings for best sleep quality. The weighted combination of temperature curves can be used as the reference personalized skin temperature curve for intelligent skin temperature regulation. In an embodiment, the weights are normalized, i.e. the sum of the weights is one. In another embodiment high rated recordings can have a high weighting factor, for example, up to three, while low weighted recordings would have a smaller weight, for example, down to zero. Next time of sleep, the sleep element may try helping to reproduce the weighted recording of skin temperature, and the reported sleep quality will weight the attempt again. When repeated over and over again, this procedure leads towards the best settings for highest subjective sleep quality.

The property recording unit can also be adapted to record a maximal and/or minimal determined property value, in particular, the maximal and/or minimal skin temperature, as the property pattern. The assignment generation unit can be adapted to determine a maximal and/or minimal determined property value, which have preferentially been assigned to good sleep quality, as a reference property pattern. The thermal control unit 204 is then preferentially adapted such that the actual skin temperature measured while the person is sleeping is above the minimal skin temperature and/or below the maximal skin temperature, wherein these minimal and maximal values have been weighted by the person as providing a good sleep quality. The sleep element 201 allows a user to adapt the temperature control to his personal preferences. The temperature control can therefore be adapted to the respective person.

Although in the above described embodiment the property recording unit is adapted to record a skin temperature of the person, in another embodiment the sleep element can be adapted to determine other properties of the person like a movement of the person and the property recording unit can be adapted to record the other properties, for example, a movement of the person by recording a corresponding movement signal. The thermal energy control unit can then be adapted to regulate the temperature of the person, for example, such that an actually measured property pattern defined by actually determined movements of the person meet a reference property pattern, which has been determined by combining several recorded property patterns to which a sleep quality has been assigned, as good as possible.

The sleep element 201 can be adapted to adjust its set point and fluctuation over time in accordance with physiological parameters of a particular person, i.e. in accordance with the recorded properties of the person. Preferentially, the sleep element 201 is adapted to adjust its set point and fluctuation over time in accordance with skin temperature fluctuations of a particular person. The sleep element 201 provides a learning algorithm which can be used to quickly personalize the sleep element to a particular person. In an embodiment, the learning period for recording property patterns which are preferentially combined for determining a reference property pattern is about one to two weeks. The sleep element 201 can be adapted to adjust its set point and fluctuation over time in accordance with physiological parameters of a particular person to achieve higher sleep quality.

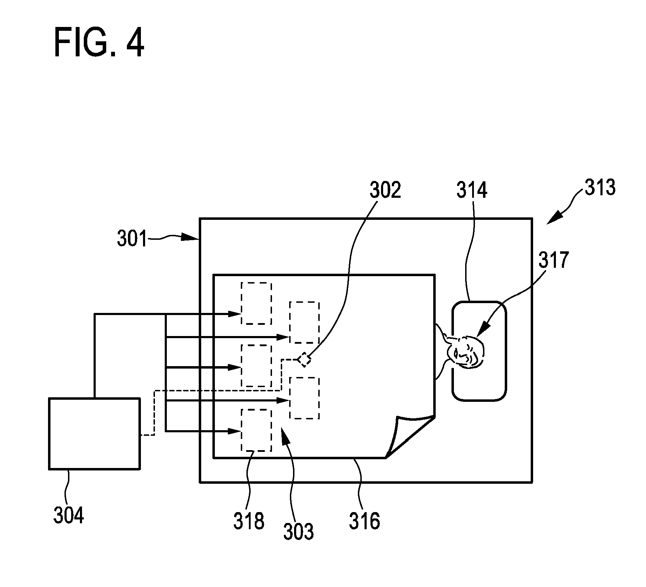

Fig. 4 shows schematically and exemplary a further embodiment of a sleep element. The sleep element 301 being an electrical blanket is located on a mattress of a bed 313. A person 317 is lying on the electrical blanket 301. The head of the person 317 is located on a pillow 314 and the person 317 is covered by a covering blanket 316. The sleep element 301, i.e. in this embodiment, the underblanket 301, comprises a property determination unit 302 being, for example, a skin temperature sensor or an accelerometer. The property determination unit 302 is embedded in the underblanket 301. The underblanket 301 comprises pockets 318 in which thermo-electric elements are inserted which form a thermal energy unit 303. In other embodiments, other elements can be used for attaching, in particular, inserting the thermo-electrical elements into the sleep element. The sleep element 301 further comprises a thermal energy control unit 304 for controlling the thermal energy unit 303 depending on the determined property of the person, i.e. e.g. depending on the measured skin temperature or a measured acceleration. The thermo-electric elements are adapted for electrically transferring thermal energy to and away from the person 317 by heating and cooling the person 317, wherein for switching from heating to cooling or vice versa the direction of the current flowing through the thermo-electric elements is modified by the thermal energy control unit 304 being preferentially a power supply like a voltage source. Switching of the electrical current to one direction provides a cooling of the person and switching of the current to another direction provides heating of the person.

Although in the above described embodiments, the sleep element is preferentially an underblanket, in other embodiments the sleep element can also be another element for being used within a bed like a covering blanket, a mattress, a pillow et cetera.

Also the other described embodiments of the sleep element can comprise pockets for holding the thermo-electric elements.

In the following an embodiment of a sleep method for improving the sleep of a person is exemplarily described with reference to a flowchart shown in Fig. 4.

In step 301, a sleep element is provided for being used within a bed. Preferentially, the sleep element is a blanket, in particular, an underblanket, which is placed on a mattress of the bed.

In step 302, a property of a person who is lying in the bed is determined by a property determination unit, for example, a moving signal is generated by using a moving sensor like an accelerometer or by using electrically conductive wires of a thermal energy unit within the sleep element, wherein an electrical property of these wires is measured as a movement signal, and by determining a comfort level depending on the generated movement signal. The determined property can also directly be the movement signal or another property like the skin temperature of the person or the heart beat of the person.

In step 303, thermal energy is transferred to or away from the person by a thermal energy unit of the sleep element, wherein the transferring of the thermal energy to or away from the person is controlled depending on the determined property of the person by a thermal energy control unit of the sleep element.

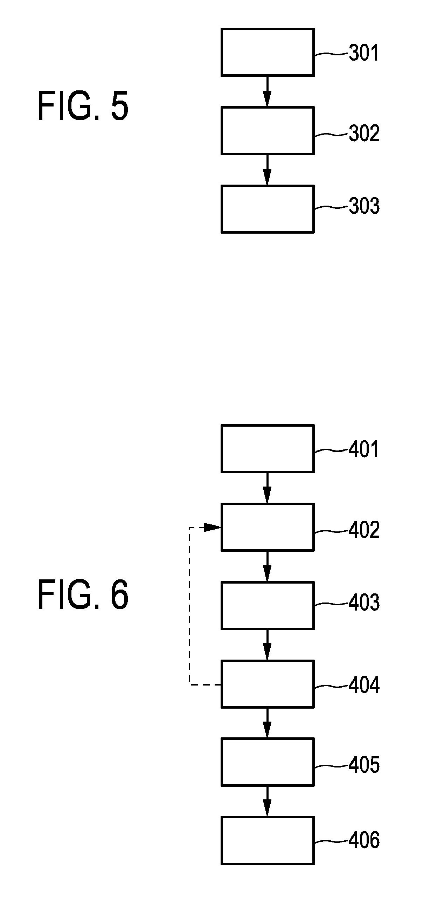

In the following a further embodiment of a sleep method for improving the sleep of a person is exemplarily described with reference to a flowchart shown in Fig. 5.

In step 401 a sleep element is provided for being used within a bed. In this embodiment, the sleep element 201 shown in Fig. 3 is provided in step 401. In step 402 a property of a person, in this embodiment, the skin temperature of the person, is determined by a property determination unit being, in this embodiment, a skin temperature sensor. The determined property of the person measured over time while the person is sleeping is recorded in a property recording unit.

In step 403, after the person has waken up, the person rates the sleep quality by using a user interface. In particular, the user selects between good sleep quality, mid-level sleep quality and bad sleep quality. In step 404, assignments between the recorded property pattern defined by the determined property over time and the selected sleep quality are generated. Steps 402 to 404 can be repeated several times, i.e. for several sleeps of the person.

In step 405 recorded property patterns which have been assigned to good sleep quality are combined to a reference property pattern. In another embodiment, also other recorded property patterns, which have not been assigned to good sleep quality can be used for generating a combined recorded property pattern being the reference property pattern. In this case, the recorded property patterns being assigned to good sleep quality receive a larger weight than recorded property patterns which have been assigned to mid-level or bad sleep quality. Moreover, preferentially, a recorded property pattern assigned to mid-level sleep quality receive a larger weight than a recorded property pattern assigned to bad sleep quality. Steps 402 to 405 can be regarded as a training method for training the sleep element such that it is adapted to a particular person.

If the person is sleeping again, in step 406 the thermal energy unit is controlled such that a deviation between actual determined properties of the person forming an actual property pattern and the reference property pattern is reduced.

Although in the above described embodiments the determined property of the person is preferentially the skin temperature or a comfort level of the person determined based on a movement of the person, in other embodiments other properties of the person can be determined like heart rate, skin conductance, electroencephalography signals, electrocardiogram signals, et cetera. Moreover, the generated movement signal can directly be regarded as the determined property of the person without determining a comfort level.

The above described sleep elements are preferentially used to improve the sleep quality of consumers, wherein unobtrusive and robust technical elements are used.

The above described sleep elements which allow to generate a movement signal can be used to utilize the activity level of a sleeping person in bed to control the heating level of the respective sleep element, in particular, of an electrical blanket. An increased activity level of the sleeping person can be interpreted as an indicator of thermal discomfort, which can be reduced by adjusting the heating of the sleep element.

Along with the increase of the life tempo, people do recognize more and more the importance of good sleep for their life. This is indicated by the growing number of the patients asking for medical help to treat insomnia, restless leg syndromes, apnea, snoring, and many other sleep disorders that were not recognized as the illness in earlier times of humankind. Even normal healthy people (sometimes just being not diagnosed patients) feel the need to improve their sleep after having stressful dynamical life, travelling across the time zones, or lacking the normal daylight due to their lifestyle, or having small babies, or getting older, or having menopauses, or many other reasons. Many of these reasons do, indeed, prevent the good sleep quality.

There are many aspects of the sleep that play a role. The sleep element in accordance with the invention refers to the thermoregulation aspect of the sleep. Two main temperatures are playing role in day-night rhythms of the humans: skin and core body temperatures. Both of them have the cyclic nature and are controlled by human physiology and consciously, when awake. In sleep, the things are more complicated. The typical heat production of the human body varies cyclically through the night from ∼100W in the evening to <50 W in the midnight and to ∼100 W again in the morning. Paradoxically, at the lowest heat production moment there should be the highest skin temperature for optimal sleep. So, historically, people cover themselves with blankets to compensate smaller heat production. Another solution that people use for ages is the heated objects put in bed: water bags, bottles, et cetera. The sleep element in accordance with the invention can be used for regulate the temperature of the sleeping person such that the sleep is effectively improved.

Although the above described embodiments comprise partly different elements, the elements of the different embodiments can be combined to a further embodiment of the invention. For example, several sensors for measuring a property of a person can be used in a single sleep element, in particular, in a single blanket. Moreover, not only the embodiment described above with reference to Fig. 1, but also further embodiments of the invention can comprise a temperature sensor being used for preventing overheating of the respective sleep element.

Although in the above described embodiments a heating wire is mainly used as a thermal energy unit, in other embodiments other thermal energy units can be used. For example, a thermo-electric element can be used which allows cooling and heating the person, wherein for switching between cooling and heating the direction of the current flowing through the thermo-electric element is modified.

The sleep element can be used for heating or cooling of a person for thermo regulation and better sleep quality. Another possible application area is to provide comfortable cooling in a hot environment and to provide heating in a cold environment. Another possible application area is the clinical assessment of patients with hypothermia or hyperthermia symptoms or the clinical assessment of patients with other symptoms.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Determinations like the determination of a property of the person, in particular, of a comfort level of a person, performed by one or several units or devices can be performed by any other number of units or devices. The determinations and/or the control of a sleep element in accordance with a sleep method can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a sleep element like a heating blanket for improving the sleep of a person. The sleep element comprises a property determination unit for determining a property of a person, a thermal energy unit for transferring thermal energy to or away from the person, and a thermal energy control unit for controlling the thermal energy unit depending on the determined property of the person. Since the sleep element comprises the property determination unit, the thermal energy unit, and the thermal energy control unit, the temperature of the person can be controlled depending on a determined property of the person, while the person is sleeping, such that the sleep of the person is improved. Moreover, the temperature control can simply be used within a bed by placing the sleep element in the bed. The handling of the temperature control is therefore very simple.

## Claims

1. A sleep element for improving the sleep of a person, the sleep element (1; 101; 201; 301) being adapted to be used within a bed and comprising:
- a property determination unit (2, 5; 103, 105; 202; 302) for determining a property of a person,
- a thermal energy unit (3; 103; 203; 303) for transferring thermal energy to or away from the person,
- a thermal energy control unit (4; 104; 204; 304) for controlling the thermal energy unit depending on the determined property of the person.

2. The sleep element as defined in claim 1, wherein the property determination unit (2; 103; 202; 302) and the thermal energy unit (3; 103; 203; 303) are integrated within the sleep element (1; 101, 201; 301).

3. The sleep element as defined in claim 1, wherein the property determination unit (2, 5; 102, 103, 105) comprises a moving sensor (2; 102, 103) for generating a movement signal being indicative of a movement of the person and a comfort level determining unit (5; 105) for determining a comfort level of the person as the property of the person depending on the generated movement signal.

4. The sleep element as defined in claim 3, wherein the comfort level determining unit (5; 105) is adapted to provide movement - comfort level assignments between movement signals and comfort levels and to determine a comfort level depending on the generated movement signal based on the provided movement - comfort level assignments.

5. The sleep element as defined in claim 4, wherein the comfort level determining unit (5; 105) is adapted to provide a movement - comfort level assignment of a movement signal to a comfort level indicating an uncomfortable condition by providing an uncomfortable threshold, wherein the comfort level determining unit (5; 105) is adapted to determine the comfort level indicating an uncomfortable condition, if the generated movement signal exceeds the uncomfortable threshold.

6. The sleep element as defined in claim 3, wherein the thermal control unit (4; 104) is adapted to determine a thermal energy pattern defining the transferring of thermal energy to or away from the person depending on the determined comfort level and to control the thermal energy unit (3; 103) such that the transferring of thermal energy to or away from the person is performed depending on the determined thermal energy pattern.

7. The sleep element as defined in claim 6, wherein the thermal energy control unit (4; 104) is adapted to provide comfort level - thermal energy assignments between comfort levels and thermal energy patterns and to determine a thermal energy pattern depending on the determined comfort level based on the comfort level - thermal energy assignments.

8. The sleep element as defined in claim 6, wherein the thermal energy control unit (4; 104) is adapted to determine a thermal energy pattern defining an additional transfer of thermal energy to the person, if the comfort level determining unit (5; 105) determines a comfort level indicating an uncomfortable condition.

9. The sleep element as defined in claim 1, wherein the thermal energy unit (3; 103; 303) comprises a thermo-electric element for electrically transferring thermal energy to or away from the person by heating or cooling the person.

10. The sleep element as defined in claim 9, wherein the thermal energy unit (103) comprises an electrically conducting element, wherein the property determining unit (105) is adapted to measure an electrical property of the electrically conducting element (103) and to determine a property of the person depending on the measured electrical property.

11. The sleep element as defined in claim 1, wherein the sleep element (1) further comprises a temperature sensor (6) for measuring the temperature of the sleep element (1), wherein the thermal energy control unit (4) is adapted to control the thermal energy unit (3) such that the thermal energy transferred to the person is lowered, if the temperature sensor (6) measures a temperature exceeding a temperature threshold.

12. The sleep element as defined in claim 1, wherein the sleep element further comprises:
- a property recording unit (207) for recording properties of the person, which have been determined during sleeping, the recorded properties of the person forming a recorded property pattern,
- a user interface (208) for allowing the person to select between at least good sleep quality and bad sleep quality,
- an assignment generation unit (212) for generating assignments between the recorded property pattern and the selected sleep quality and for determining a reference property pattern depending on the generated assignments,
wherein the thermal control unit (204) is adapted to control the thermal energy unit (203) such that a deviation of actually determined properties of the person forming an actual property pattern and the reference property pattern is reduced.

13. A bed comprising the sleep element as defined in claim 1.

14. A sleep method for improving the sleep of a person, wherein a sleep element (1; 101; 201; 301) for being used within a bed as defined in claim 1 is provided and wherein the sleep method comprises following steps:
- determining a property of a person by the property determination unit (2, 5; 103, 105; 202; 302),
- transferring thermal energy to or away from the person by the thermal energy unit (4; 104; 204; 304),
wherein the transferring of the thermal energy to or away from the person is controlled depending on the determined property of the person by the thermal energy control unit (4; 104; 204; 304).

15. A sleep computer program for improving the sleep of a person, the sleep computer program comprising program code means for causing a sleep element as defined in claim 1 to carry out the steps of the sleep method as defined in claim 14, when the sleep computer program is run on a computer controlling the sleep element.
